# FASCICULE DE BREVET EUROPEEN

(11) **EP 0 635 497 B1**
(45) Date de publication et mention de la délivrance du brevet: **20.05.1998**
(21) Numéro de dépôt: 94401549.4
(22) Date de dépôt: 06.07.1994
(51) Int. Cl.: C07D 235/02, C07D 263/52, C07D 491/107, C07D 495/10, A61K 31/415, A61K 31/42

(54) **Nouveaux dérivés de benzospiroalcène, leur procédé de préparation et les compositions pharmaceutiques qui les contiennent**
Benzospiroalken-Derivate, Verfahren zu ihrer Herstellung und diese enthaltende pharmazeutische Zusammensetzungen
Benzospiroalkene derivatives, process for their preparation and pharmaceutical compostions containing them

(30) Priorité: 20.07.1993 FR 9308861
(43) Date de publication de la demande: 25.01.1995
(73) Titulaire: ADIR ET COMPAGNIE, 92415 Courbevoie Cédex (FR)
(72) Inventeur: Cordi, Alex, F-92150 Suresnes (FR); Lacoste, Jean-Michel, F-92310 Sevres (FR); Laubie, Michel, F-92420 Vaucresson (FR); Verbeuren, Tony, F-78540 Vernouillet (FR); Descombes, Jean-Jacques, F-93360 Neuilly-Plaisance (FR)

(56) Documents cités:
- JOURNAL OF MEDICINAL CHEMISTRY., vol.21, no.6, 1978, WASHINGTON US pages 585 - 587 P. A. CROOKS ET AL 'Synthesis of spiro(tetralin-2,2'-pyrrolidine) and spiro(indan-2,2'-pyrrolidine) derivatives as potential analgesics'
- JOURNAL OF THE CHEMICAL SOCIETY, PERKIN TRANSACTIONS 1., no.11, 1979, LETCHWORTH GB pages 2719 - 2726 P. A. CROOKS ET AL 'Synthesis of 5-hydroxy and 5,6-dihydroxy-derivatives of spiro(indane-2,2'-pyrrolidine), rigid analogues of tyramine and dopamine respectively'

## Description

J. Med. Chem., 21, 585-587, 1978 et J. Chem. Soc. Perkin Transactions 1, 2719-2726, 1979 décrivent des composés spiro[(pyrrolidine)-2:2'(1',2',3',4'-tetrahydronaphtalène)] et spiro[(pyrrolidine)-2:2'-(indane)] qui ont une activité analgésique.

La présente invention concerne des benzospiroalcènes, leur procédé de préparation et les compositions pharmaceutiques qui les contiennent, ainsi que leur utilisation en tant qu'agonistes partiels α₁ et α₂ adrénergiques.

Le système nerveux adrénergique joue un rôle important à plusieurs niveaux, par exemple artériel, veineux, cardiaque, rénal et au niveau du système nerveux autonome central et périphérique. Dès lors, les produits capables d'intéragir avec les récepteurs adrénergiques peuvent induire un grand nombre de réponses physiologiques comme la vasoconstriction, la vasodilatation, l'augmentation ou la diminution du rythme cardiaque, la variation de la force de contraction du muscle cardiaque et des activités métaboliques, comme l'ont indiqué P. TIMMERMANS et coll. dans "Comprehensive Medicinal Chemistry" (Vol. III, p. 134-185 - C. HANSH Editor, Pergamon, Oxford, 1990). Différents composés adrénergiques ont été utilisés dans le passé pour modifier ces réponses physiologiques ou d'autres.

La stimulation adrénergique dans le système nerveux périphérique est thérapeutiquement utile lorsqu'une constriction vasculaire a lieu telle que dans les congestions nasales, optiques ou ophtalmiques et dans l'inflammation. Une interaction antagoniste peut moduler l'activité des neurones adrénergiques et l'équilibre hémodynamique ce qui est utile dans de multiples indications cardiovasculaires telles que l'hypertension, l'arrêt cardiaque et une variété de condition de spasme vasculaire ainsi que pour remédier à des épisodes d'impuissance masculine légère.

Dans le système nerveux central, la stimulation adrénergique est particulièrement utile pour induire de la sédation et de la diurèse et dans le traitement de l'hypertension et du comportement addictif. Par contraste, un antagoniste adrénergique peut être utile dans certains désordres psychiatriques ou neurologiques tel que la dépression ainsi que pour le maintien de l'équilibre cardiovasculaire.

Les composés décrits dans la présente invention possèdent un profil d'agonistes partiels α₁ et α₂ adrénergiques qui les rend plus particulièrement capables de diminuer la compliance veineuse sans affecter la compliance artérielle, cet effet étant favorisé par une élévation de la température. Ces propriétés très favorables indiquent que les composés de l'invention sont utiles dans le soulagement des symptômes de jambes lourdes liés à un déficit du retour veineux particulièrement au niveau des membres inférieurs (maladie veineuse). De plus, ils peuvent également être utiles, comme sympathomimétiques, dans le traitement de l'hypertension, de l'anxiété, des psychoses, du diabète, de l'impotence et de la douleur, ainsi que comme sédatifs, vasoconstricteurs, décongestionnants, hypotenseurs oculaires et pour remédier aux symptômes de l'abstinence opiacée. Enfin, les composés de l'invention sont également capables de provoquer une baisse du débit carotidien ce qui les rend également utiles dans le traitement de la migraine. Cette propriété a été montrée notamment par R. SAXENA et coll. (TIPS, Vol. 10, p. 200-204, 1989) et par M.O. den BOER et coll. (Br. J. Pharmacol., 102, 323-330, 1991). L'utilité thérapeutique des produits de l'invention se base sur leur sélectivité pour les sous-types de récepteurs adrénergiques et leur modulation sélective des fonctions adrénergiques dans différents tissus et organes.

Des agonistes partiels α adrénergiques ont déjà été décrits dans la littérature. C'est le cas plus particulièrement des composés décrits dans le brevet BE 687,657. Mais la capacité de ces composés à diminuer la compliance veineuse sans affecter la compliance artérielle est nettement moins intense que celle obtenue avec les composés de l'invention.

Plus spécifiquement la présente invention concerne les composés de formule (I) : 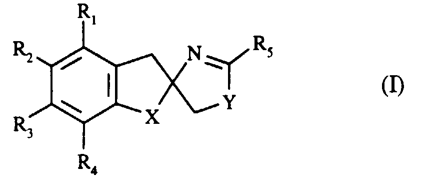 dans laquelle :
- X représente -CH₂-, -(CH₂)₂-, -CH=CH-, -O-CH₂-, -S-CH₂-, -SO-CH₂- ou -SO₂-CH₂-,
- Y représente un atome d'oxygène, de soufre, ou un groupement -NR₆-,
- R₁ représente un atome d'hydrogène ou un groupement alkyle (C₁-C₆) linéaire ou ramifié,
- R₂ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₃ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₄ représente un atome d'hydrogène (à la condition que dans ce cas R₁ représente un atome d'hydrogène), un atome d'halogène, un groupement alkyle (C₁-C₆) linéaire ou ramifié (substitué ou non par un ou plusieurs atomes d'halogène) ou un groupement hydroxy,
ou bien
- R₁ et R₂, R₂ et R₃, R₃ et R₄ ou R₄ et X forment ensemble avec les atomes de carbone qui les portent, un cycle benzénique à la condition que, dans le cas où R₁ et R₂ forment un cycle benzénique X soit différent de -CH₂- ou -(CH₂)₂-,
- R₅ représente un atome d'hydrogène ou un groupement amino (substitué ou non par un ou deux groupements alkyle (C₁-C₆) linéaire ou ramifié),
- R₆ a la même signification que R₁,
leurs isomères ainsi que leurs sels d'addition à un acide pharmaceutiquement acceptable.

Parmi les acides pharmaceutiquement acceptables on peut citer à titre non limitatif les acides chlorhydrique, bromhydrique, sulfurique, phosphorique, acétique, lactique, malonique, succinique, fumarique, tartrique, maléique, citrique, méthane sulfonique, benzène sulfonique, etc...

Parmi les isomères éventuels des composés de formule (I), on peut citer les énantiomères, les diastéréoisomères, les épimères ainsi que les tautomères.

L'invention s'étend également au procédé de préparation des composés de formule (I).

Les composés de formule (I) pour lesquels Y représente un groupement -NR₆, sont obtenus selon le procédé caractérisé en ce que l'on utilise comme produit de départ un composé de formule (II) : 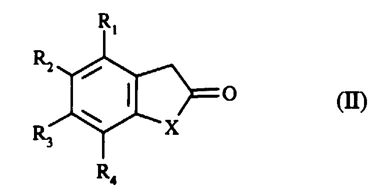 dans laquelle X, R₁, R₂, R₃ et R₄ ont la même signification que dans la formule (I)
que l'on fait réagir :
- soit avec de la benzylamine en présence d'acide paratoluènesulfonique pour conduire au composé de formule (III) : 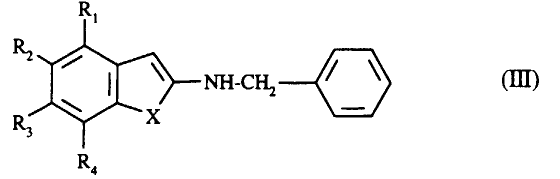 dans laquelle X, R₁, R₂, R₃ et R₄ ont la même signification que dans la formule (I)
   que l'on fait réagir, sous atmosphère inerte, avec du cyanure de triméthylsilyle en présence d'iodure de zinc,
   pour conduire au composé de formule (IV) : 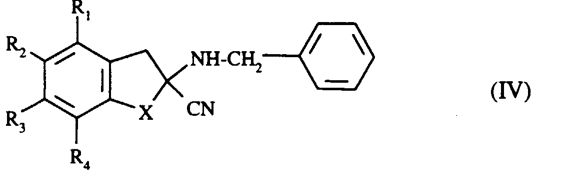 dans laquelle X, R₁, R₂, R₃ et R₄ ont la même signification que dans la formule (I),
   que l'on réduit à l'aide d'hydrure de lithium aluminium, puis par hydrogénation catalytique pour conduire au composé de formule (V) : 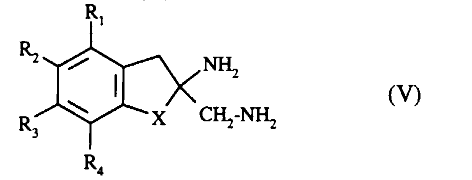 dans laquelle X, R₁, R₂, R₃ et R₄ ont la même signification que dans la formule (I),
- soit avec du cyanure de potassium en présence de chlorure d'ammonium en milieu inerte ou avec du cyanure de sodium en milieu acide, ou bien avec du cyanure de triméthylsilyle en présence d'iodure de zinc puis avec une solution alcoolique saturée en ammoniac,
   pour conduire au composé de formule (VI) : 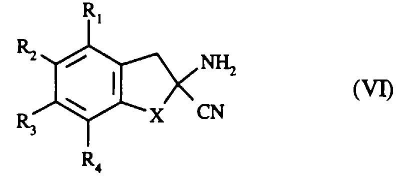 dans laquelle X, R₁, R₂, R₃ et R₄ ont la même signification que dans la formule (I),
   que l'on réduit à l'aide d'hydrure de lithium aluminium pour conduire au composé de formule (V) décrit précédemment,
composé de formule (V),
que l'on fait réagir avec de la formamidine en milieu alcoolique, un formiate d'alkyle ou avec un halogènure de cyanogène (suivie, selon la nature du composé de formule (I) que l'on souhaite obtenir, d'une réaction d'alkylation à l'aide d'un halogénure d'alkyle),
pour conduire au composé de formule (I/a), cas particulier des composés de formule (I) : 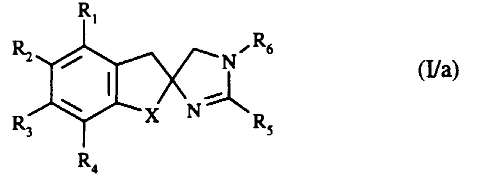 dans laquelle X, R₁, R₂, R₃, R₄, R₅ et R₆ ont la même signification que dans la formule (I)
composé de formule (I/a),
que l'on purifie, le cas échéant, selon une technique classique de purification,
dont on sépare, si on le souhaite, les isomères selon une technique classique de purification,
et que l'on transforme, éventuellement, en ses sels d'addition à un acide pharmaceutiquement acceptable.

Les composés de formule (I) pour lesquels Y représente un atome d'oxygène ou de soufre sont obtenus selon le procédé caractérisé en ce que l'on utilise comme produit de départ un composé de formule (VI) décrit précédemment,
que l'on fait réagir avec de l'acide formique en milieu anhydre saturé en acide chlorhydrique, pour conduire au composé de formule (VII) : 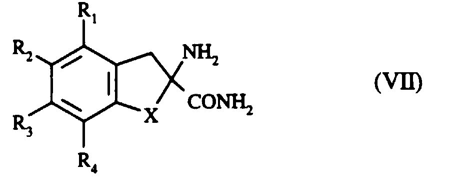 dans laquelle R₁, R₂, R₃, R₄ et X ont la même signification que dans la formule (I),
que l'on transforme en acide correspondant de formule (VIII) en milieu chlorhydrique concentré : 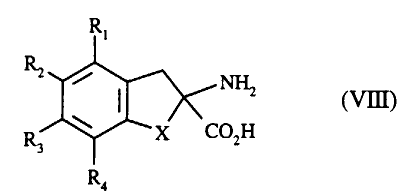 dans laquelle R₁, R₂, R₃, R₄ et X ont la même signification que dans la formule (I),
qui subit une réduction par l'hydrure de lithium aluminium en milieu inerte,
pour conduire au composé de formule (IXa) : 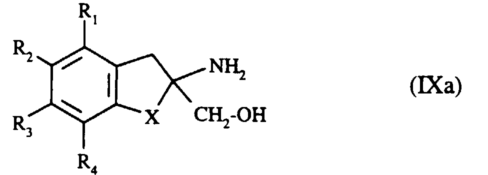 dans laquelle R₁, R₂, R₃, R₄ et X ont la même signification que dans la formule (I),
composé de formule (IXa), que l'on transforme, selon la nature des composés de formule (I) que l'on souhaite obtenir, en tosylate correspondant à l'aide d'acide p.toluènesulfonique puis que l'on fait réagir avec de la thiourée ou de l'acide thioacétique,
pour conduire, après hydrolyse, au composé de formule (IXb) : 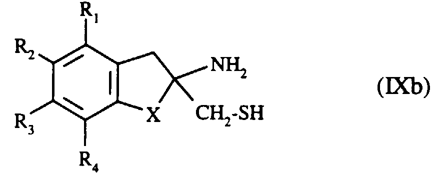 dans laquelle R₁, R₂, R₃, R₄ et X ont la même signification que dans la formule (I),
composé de formule (IXa) ou (IXb) que l'on fait réagir avec de la formamidine en milieu alcoolique, un formiate d'alkyle ou avec un halogénure de cyanogène (suivie, selon la nature du composé de formule (I) que l'on souhaite obtenir, d'une réaction d'alkylation à l'aide d'un halogénure d'alkyle),
pour conduire au composé de formule (I/b), cas particulier des composés de formule (I) : 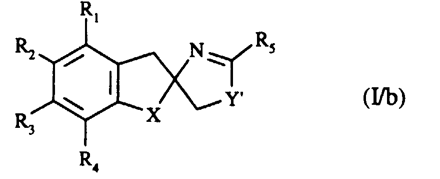 dans laquelle R₁, R₂, R₃, R₄, R₅ et X ont la même signification que précédemment et Y' représente un atome d'oxygène ou de soufre,
composé de formule (I/b)
que l'on purifie, le cas échéant, selon une technique classique de purification,
dont on sépare, si on le souhaite, les isomères selon une technique classique de purification,
et que l'on transforme, éventuellement, en ses sels d'addition à un acide pharmaceutiquement acceptable.

Lorsque les composés de formule (I) que l'on souhaite obtenir possèdent en R₂, R₃ ou R₄ un groupement hydroxy, un procédé préférentiel d'obtention de ces composés consiste à synthétiser dans un premier temps le dérivé de formule (I) possédant en R₂, R₃ ou R₄ un groupement alkoxy que l'on transforme en groupement hydroxy correspondant par action du tribromure de bore en milieu dichlorométhane.

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiées, les comprimés sublingaux, les gélules, les tablettes, les suppositoires, les crèmes, pommades, gels dermiques, etc...

La posologie varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,1 et 1000 mg pour un traitement en 1 à 3 prises par 24 heures.

Les exemples suivants illustrent l'invention. Les produits de départ utilisés sont des produits connus ou préparés selon des modes opératoires connus.

### Exemple 1: Spiro[(1,3-diazacyclopent-1-ène)-5 : 2'-(1',2',3',4'-tétrahydronaphtalène)], benzènesulfonate

### Stade A : 2-Benzylamino-3,4-dihydronaphtalène

Un mélange contenant 171 mmoles de β-tétralone, 181 mmoles de benzylamine et 100 mg d'acide p.toluènesulfonique est porté au reflux avec distillation azéotropique du mélange eau/toluène. Après 2 heures de distillation, le milieu est refroidi et filtré. Le produit attendu est obtenu sous forme d'huile après évaporation du solvant.

### Stade B : 2-Benzylamino-2-cyano-1,2,3,4-tétrahydronaphtalène

A une solution, maintenue sous azote, contenant 170 mmoles du composé obtenu au stade précédent dans 600 ml de dichlorométhane sont ajoutées successivement 170 mmoles de cyanure de triméthylsilyle et 87 mmoles d'iodure de zinc. Le mélange est agité à 20°C pendant une nuit puis lavé par de l'eau.
Après séchage et évaporation de la phase organique, on obtient le produit attendu sous forme d'huile.

### Stade C : 2-Benzylamino-2-aminométhyl-1,2,3,4-tétrahydronaphtalène

A une suspension contenant 290 mmoles d'hydrure de lithium aluminium dans 700 ml de tétrahydrofurane anhydre est additionnée goutte à goutte une solution contenant 152 mmoles du composé obtenu au stade précédent dans 200 ml de tétrahydrofurane anhydre, en maintenant la température inférieure à 30°C. Après 2 heures d'agitation, le mélange est refroidi à 0°C et hydrolysé par addition successive de 11 ml d'eau, 11 ml de soude 2N et 25 ml d'eau. La suspension est filtrée et le filtrat évaporé. Le résidu est dissous dans 400 ml d'acétate d'éthyle. Cette phase est lavée par de l'eau et extraite par de l'acide chlorhydrique 1N. Les phases aqueuses acides sont réunies , alcalinisées par de la soude à 35 % et extraites par de l'acétate d'éthyle.
La phase organique est séchée et évaporée pour conduire au produit attendu.

### Stade D : 2-Amino-2-aminométhyl-1,2,3,4-tétrahydronaphtalène

Une suspension contenant 30 mmoles du composé obtenu au stade précédent, 125 mmoles de formiate d'ammonium, 6 g de Palladium sur charbon (10 %) et 250 ml de méthanol est portée à reflux sous agitation pendant 20 minutes. Après refroidissement, filtration du catalyseur, évaporation du solvant, le produit attendu est obtenu sous forme d'huile, après purification par chromatographie sur colonne de silice en utilisant comme éluant un mélange dichlorométhane/méthanol/ammoniaque (90/10/1).

### Stade E : Spiro[(1,3-diazacyclopent-1-ène)-5 : 2'-(1',2',3',4'-tétrahydronaphtalène)],benzènesulfonate

Un mélange contenant 20 mmoles du composé obtenu au stade précédent et 20 mmoles d'acétate de formamidine dans 60 ml d'éthanol est agité à 20°C sous azote pendant 12 heures. Le solvant est évaporé et le résidu repris par 50 ml d'acide chlorhydrique 1N.
La phase acide est lavée par de l'acétate d'éthyle puis alcalinisée par de la soude à 35 %. Le mélange est extrait par de l'acétate d'éthyle. Les phases organiques réunies sont lavées par une solution aqueuse de chlorure de sodium, évaporées et conduisent à un résidu solide. Ce résidu est dissous dans 50 ml d'éthanol puis traité par un équivalent d'acide benzène sulfonique dissous dans 20 ml d'éthanol.
Le produit attendu est obtenu après évaporation du solvant et cristallisation dans un mélange éthanol/éther.

Point de fusion : 124°C

| Microanalyse élémentaire : | | | | |
|---|---|---|---|---|
| | C % | H % | N % | S % |
| calculé | 62,77 | 5,85 | 8,13 | 9,31 |
| trouvé | 63,06 | 6,03 | 8,23 | 9,52 |

Les exemples 2 à 12 ont été obtenus selon le procédé décrit dans l'exemple 1 en utilisant les produits de départ correspondants.

### Exemple 2: Spiro[(1,3-diazacyclopent-1-ène)-5 : 2',(7'-trifluorométhyl-1',2',3',4'-tétrahydronaphtalène)], fumarate

Point de fusion : 168-170°C

| Microanalyse élémentaire : | | | |
|---|---|---|---|
| | C % | H % | N % |
| calculé | 55,14 | 4,63 | 7,56 |
| trouvé | 55,02 | 4,63 | 7,68 |

### Exemple 3: Spiro[(1,3-diazacyclopent-1-ène)-5 : 2'-(6'-hydroxy-7'-méthoxy-1',2',3',4'-tétrahydronaphtalène)], fumarate

Point de fusion : 219-222°C

| Microanalyse élémentaire : | | | |
|---|---|---|---|
| | C % | H % | N % |
| calculé | 58,61 | 5,79 | 8,04 |
| trouvé | 58,74 | 6,00 | 8,00 |

### Exemple 4: Spiro[(1,3-diazacyclopent-1-ène)-5 : 2'-(5',7'-diméthyl-1',2',3',4'-tétrahydronaphtalène)], fumarate

Point de fusion : 208-212 °C

| Microanalyse élémentaire : | | | |
|---|---|---|---|
| | C % | H % | N % |
| calculé | 69,37 | 7,24 | 9,87 |
| trouvé | 69,29 | 7,33 | 10,03 |

### Exemple 5: Spiro[(1,3-diazacyclopent-1-ène)-5 : 2'-(6'-isopropyloxy-1',2',3',4'-tétrahydronaphtalène)], fumarate

Point de fusion : 172-177°C

| Microanalyse élémentaire : | | | |
|---|---|---|---|
| | C % | H % | N % |
| calculé | 63,31 | 6,71 | 7,77 |
| trouvé | 63,37 | 6,68 | 7,88 |

### Exemple 6: Spiro[(1,3-diazacyclopent-1-ène)-5 : 2'-(5',7'-diméthoxy-1',2',3',4'-tétrahydronaphtalène)], fumarate

Point de fusion : 150-154°C

| Microanalyse élémentaire : | | | |
|---|---|---|---|
| | C % | H % | N % |
| calculé | 59,66 | 6,12 | 7,73 |
| trouvé | 59,38 | 5,87 | 7,80 |

### Exemple 7: Spiro[(1,3-diazacyclopent-1-ène)-5 : 2'-(5',6'-diméthoxy-1',2',3',4'-tétrahydronaphtalène)], fumarate

Point de fusion: 176-178°C

| Microanalyse élémentaire : | | | |
|---|---|---|---|
| | C % | H % | N % |
| calculé | 59,66 | 6,12 | 7,73 |
| trouvé | 59,49 | 6,14 | 7,72 |

### Exemple 8: Spiro[(1,3-diazacyclopent-1-ène)-5 : 2'-(6'-méthyl-1',2',3',4'-tétrahydronaphtalène)], fumarate

Point de fusion : 170-174°C

| Microanalyse élémentaire : | | | |
|---|---|---|---|
| | C % | H % | N % |
| calculé | 64,54 | 6,37 | 8,85 |
| trouvé | 64,84 | 6,30 | 8,98 |

### Exemple 9: Spiro[(1,3-diazacyclopent-1-ène)-5 : 2'-(5'-méthoxy-7'-méthyl-1',2',3',4'-tétrahydronaphtalène)], fumarate

Point de fusion : 178-181°C

| Microanalyse élémentaire : | | | |
|---|---|---|---|
| | C % | H % | N % |
| calculé | 62,48 | 6,33 | 7,95 |
| trouvé | 62,42 | 6,40 | 8,09 |

### Exemple 10: Spiro[(1,3-diazacyclopent-1-ène)-5 : 2'-(5'-trifluorométhyl indane)], fumarate

Point de fusion : 175-177°C

| Microanalyse élémentaire : | | | |
|---|---|---|---|
| | C % | H % | N % |
| calculé | 53,94 | 4,24 | 13,42 |
| trouvé | 53,71 | 4,07 | 13,50 |

### Exemple 11: Spiro[(1,3-diazacyclopent-1-ène)-5 : 2'-(5'-méthoxyindane)], fumarate

Point de fusion : 227-230°C

| Microanalyse élémentaire : | | | |
|---|---|---|---|
| | C % | H % | N % |
| calculé | 57,44 | 5,36 | 7,44 |
| trouvé | 57,22 | 5,51 | 7,59 |

### Exemple 12: Spiro[(1,3-diazacyclopent-1-ène)-5 : 3'-(7'-méthoxychromane)], fumarate

Point de fusion : 186-190°C

| Microanalyse élémentaire : | | | |
|---|---|---|---|
| | C % | H % | N % |
| calculé | 57,48 | 5,43 | 8,38 |
| trouvé | 57,60 | 5,27 | 8,41 |

### Exemple 13: Spiro[(1,3-diazacyclopent-1-ène)-5 : 2'-1',2',3',4'-tetrahydronaphtalène)], D(-) tartrate, isomère α

Le composé de l'exemple 1 est dédoublé au moyen d'acide L-dibenzoyl tartrique par recristallisations successives dans l'éthanol. La pureté énantiomérique est vérifiée par chromatographie sur colonne chirale α₁-AGP en utilisant comme éluant un mélange Na₂HPO₄ aq. 0,01 M/NaH₂PO₄ aq. 0,01 M/n-propanol (60/40/1). Le sel pur est alors partagé entre la soude 1N et le dichlorométhane. La phase aqueuse est extraite par du dichlorométhane. Les phases organiques sont séchées et évaporées. Le résidu solide est dissous dans de l'éthanol, un équivalent d'acide D(-) tartrique est ajouté et l'ensemble est porté à reflux. Le produit attendu est obtenu par refroidissement sous forme d'un solide blanc.

Point de fusion : 185-186 °C

| Mikroanalyse élémentaire : | | | |
|---|---|---|---|
| | C % | H % | N % |
| calculé | 57,14 | 5,99 | 8,33 |
| trouvé | 57,08 | 5,74 | 8,22 |

### Exemple 14: Spiro[(1,3-diazacyclopent-1-ène)-5 : 2'-(1',2',3',4'-tétrahydronaphtalène)], L(+) tartrate, isomère β

Le produit attendu est obtenu selon le procédé décrit dans l'exemple 13 à partir du composé de l'exemple 1 et d'acide L(+) tartrique.

### Exemple 15: Spiro[(1,3-diazacyclopent-1-ène)-5 : 2'-(6',7'-dichloro-1',2',3',4'-tétrahydronaphtalène)], fumarate

### Stade A : 2-Amino-2-cyano-7-chloro-1,2,3,4-tétrahydronaphtalène

A une solution fortement agitée et maintenue sous azote contenant 138 mmoles de 7-chloro-3,4-dihydronaphtalèn-2-(1H)-one dans 170 ml de méthanol et 85 ml d'eau, sont ajoutées successivement 141 mmoles de cyanure de potassium et 145 mmoles de chlorure d'ammonium. Après 48 heures d'agitation à 20 °C, le mélange est concentré. Le résidu est repris par de l'acétate d'éthyle. Cette phase organique est lavée à l'eau puis extraite par de l'acide chlorhydrique 1N. Les phases acides sont alcalinisées par de la soude à 35 % et extraites par de l'acétate d'éthyle.
Les phases organiques sont séchées, évaporées et conduisent au produit attendu sous forme d'huile.

### Stade B : 2-Amino-2-aminométhyl-7-chloro-1,2,3,4-tétrahydronaphtalène

A une suspension contenant 175 mmoles d'hydrure de lithium aluminium dans 250 ml de tétrahydrofurane anhydre est additionnée, goutte à goutte, à une température inférieure à 20°C, une solution contenant 76 mmoles du produit obtenu au stade précédent dans 100 ml de tétrahydrofurane anhydre. Après une heure d'agitation, le mélange est refroidi à 0°C et hydrolysé par addition de 6,5 ml de soude 2N puis 14 ml d'eau. La suspension résultante est filtrée et le filtrat évaporé. Le résidu est dissous dans de l'acétate d'éthyle et cette solution est lavée par de l'eau, extraite par de l'acide chlorhydrique 1N.
Les phases acides sont alors alcalinisées par de la soude à 35 % et extraites par de l'acétate d'éthyle.
Après séchage et évaporation, on obtient le produit attendu sous forme d'huile.

### Stade C : Spiro[(1,3-diazacyclopent-1-ène)-5 : 2'-(6',7'-dichloro-1',2',3',4'-tétrahydronaphtalène)], fumarate

Un mélange contenant 26 mmoles du composé obtenu au stade précédent, 29 mmoles d'acétate de formamidine dans 100 ml d'éthanol est agité, à 20°C, sous atmosphère d'azote, pendant 12 heures. Après évaporation du solvant, le résidu est repris par de l'acide chlorhydrique 1N. La phase acide est lavée par de l'acétate d'éthyle, alcalinisée par de la soude à 35 % et extraite par de l'acétate d'éthyle.
Les phases organiques réunies sont lavées par une solution saturée de chlorure de sodium puis évaporées. Le résidu solide est repris par de l'éthanol traité par un équivalent d'acide fumarique dissous dans 30 ml d'éthanol.
Après évaporation du solvant, le produit attendu est obtenu par recristallisation du résidu dans de l'éthanol.

Point de fusion : 138-140°C

| Microanalyse élémentaire : | | | | |
|---|---|---|---|---|
| | C % | H % | N % | Cl % |
| calculé | 57,06 | 5,09 | 8,32 | 10,53 |
| trouvé | 57,15 | 5,18 | 8,31 | 10,43 |

Les exemples 16 à 28 ont été obtenus selon le procédé décrit dans l'exemple 15 en utilisant les produits de départ correspondants.

### Exemple 16 : Spiro[(1,3-diazacyclopent-1-ène)-5 : 2'-(7'-méthoxy-1',2',3',4'-tétrahydronaphtalène)], fumarate

Point de fusion : 188-190°C

| Microanalyse élémentaire : | | | |
|---|---|---|---|
| | C % | H % | N % |
| calculé | 61,44 | 6,07 | 8,43 |
| trouvé | 61,49 | 6,02 | 8,26 |

### Exemple 17: Spiro[(1,3-diazacyclopent-1-ène)-5 : 2'-(5',7'-dichloro-1',2',3',4'-tétrahydronaphtalène)], fumarate

Point de fusion : 204-205°C

| Microanalyse élémentaire : | | | | |
|---|---|---|---|---|
| | C % | H % | N % | Cl % |
| calculé | 51,77 | 4,34 | 7,55 | 19,10 |
| trouvé | 51,84 | 4,34 | 7,62 | 18,80 |

### Exemple 18: Spiro[(1,3-diazacyclopent-1-ène)-5 : 2'-(6',7'-dichloro-1',2',3',4'-tétrahydronaphtalène)], fumarate

Point de fusion : 214-216°C

| Microanalyse élémentaire : | | | | |
|---|---|---|---|---|
| | C % | H % | N % | Cl % |
| calculé | 51,77 | 4,34 | 7,55 | 19,10 |
| trouvé | 51,56 | 4,22 | 7,45 | 19,03 |

### Exemple 19: Spiro[(1,3-diazacyclopent-1-ène)-5 : 2'-(6',7'-diméthoxy-1',2',3',4'-tétrahydronaphtalène)], fumarate

Point de fusion : 138-140°C

| Microanalyse élémentaire : | | | |
|---|---|---|---|
| | C % | H % | N % |
| calculé | 59,66 | 6,12 | 7,77 |
| trouvé | 59,20 | 6,07 | 7,58 |

### Exemple 20: Spiro[(1,3-diazacyclopent-1-ène)-5 : 2'-(6'-chloro-1',2',3',4'-tétrahydronaphtalène)], fumarate

Point de fusion : 201-203°C

| Microanalyse élémentaire : | | | | |
|---|---|---|---|---|
| | C % | H % | N % | Cl % |
| calculé | 57,06 | 5,09 | 8,32 | 10,53 |
| trouvé | 57,12 | 4,86 | 8,20 | 10,47 |

### Exemple 21: Spiro[(1,3-diazacyclopent-1-ène)-5 : 2'-(6'-méthoxy-1',2',3',4'-tétrahydronaphtalène)], fumarate

Point de fusion : 172-174 °C

| Microanalyse élémentaire : | | | |
|---|---|---|---|
| | C % | H % | N % |
| calculé | 61,44 | 6,07 | 8,43 |
| trouvé | 61,35 | 5,65 | 8,41 |

### Exemple 22: Spiro[(1,3-diazacyclopent-1-ène)-5 : 2'-(5'-chloro-1',2',3',4'-tétrahydronaphtalène)], fumarate

Point de fusion : 204-206°C

| Microanalyse élémentaire : | | | | |
|---|---|---|---|---|
| | C % | H % | N % | Cl % |
| calculé | 57,06 | 5,09 | 8,32 | 10,53 |
| trouvé | 57,26 | 5,22 | 8,27 | 10,48 |

### Exemple 23: Spiro[(1,3-diazacyclopent-1-ène)-5 : 2'-(7'-fluoro-1',2',3',4'-tétrahydronaphtalène)], fumarate

Point de fusion : 196-200°C

| Microanalyse élémentaire : | | | |
|---|---|---|---|
| | C % | H % | N % |
| calculé | 63,15 | 5,67 | 10,23 |
| trouvé | 63,16 | 5,65 | 10,20 |

### Exemple 24: Spiro[(1,3-diazacyclopent-1-ène)-5 : 2'-(7'-méthyl-1',2',3',4'-tétrahydronaphtalène)], fumarate

Point de fusion : 210-214°C

| Microanalyse élémentaire : | | | |
|---|---|---|---|
| | C % | H % | N % |
| calculé | 68,53 | 6,87 | 10,38 |
| trouvé | 69,02 | 6,88 | 10,75 |

### Exemple 25: Spiro[(1,3-diazacyclopent-1-ène)-5 : 2'-(5',7'-difluoro-1',2',3',4'-tétrahydronaphtalène)], fumarate

Point de fusion : 118-124°C

| Microanalyse élémentaire : | | | |
|---|---|---|---|
| | C % | H % | N % |
| calculé | 60,00 | 5,03 | 9,99 |
| trouvé | 59,68 | 4,84 | 9,99 |

### Exemple 26: Spiro[(1,3-diazacyclopent-1-ène)-5 : 3'-(chromane)], fumarate

Point de fusion : 175-176°C

| Microanalyse élémentaire : | | | |
|---|---|---|---|
| | C % | H % | N % |
| calculé | 59,20 | 5,31 | 9,21 |
| trouvé | 58,79 | 5,45 | 8,16 |

### Exemple 27: Spiro[(1,3-diazacyclopent-1-ène)-5 : 3'-(thiochromane)], fumarate

Point de fusion : 176-178°C

| Microanalyse élémentaire : | | | | |
|---|---|---|---|---|
| | C % | H % | N % | S % |
| calculé | 56,24 | 5,03 | 8,74 | 10,01 |
| trouvé | 56,22 | 5,16 | 8,82 | 9,94 |

### Exemple 28: Spiro[(1,3-diazacyclopent-1-ène)-5 : 2'-(indane)], chlorhydrate

Point de fusion : 210°C

| Microanalyse élémentaire : | | | | |
|---|---|---|---|---|
| | C % | H % | N % | Cl % |
| calculé | 63,31 | 6,28 | 13,42 | 16,99 |
| trouvé | 63,15 | 6,26 | 13,31 | 17,06 |

### Exemple 29: Spiro[(1,3-diazacyclopent-1-ène)-5 : 2'-(6'-méthoxy-1',2',3',4'-tétrahydronaphtalène)], fumarate, hydrate, isomère α

Le produit attendu a été obtenu par dédoublement du composé décrit dans l'exemple 21 au moyen d'acide R(-)campho-10-sulfonique. Le sel de l'énantiomère pur ainsi obtenu a été neutralisé puis salifié a nouveau à l'aide d'acide fumarique.

La pureté énantiomérique a été vérifiée par chromatographie liquide sur colonne DIACELOD en utilisant comme éluant un mélange isopropanol/n-heptane/diéthylamine (25/75/0,08).

Point de fusion : 105-107°C

| Microanalyse élémentaire : | | | |
|---|---|---|---|
| | C % | H % | N % |
| calculé | 58,58 | 6,33 | 8,00 |
| trouvé | 58,25 | 6,50 | 7,82 |

### Exemple 30: Spiro[(1,3-diazacyclopent-1-ène)-5 : 2'-(6'-méthoxy-1',2',3',4'-tétrahydronaphtalène)], fumarate, hydrate isomère β

Le produit attendu a été isolé selon le procédé décrit dans l'exemple 29 après dédoublement du composé de l'exemple 21 par de l'acide S(+) campho-10-sulfonique.

Point de fusion : 105-107°C

| Microanalyse élémentaire : | | | |
|---|---|---|---|
| | C % | H % | N % |
| calculé | 58,28 | 6,33 | 8,00 |
| trouvé | 57,83 | 6,38 | 7,87 |

### Exemple 31: Spiro[(1,3-diazacyclopent-1-ène)-5 : 2'-(2',3'-dihydrophénalène)], 1/2 fumarate

### Stade A : 2-Amino-2-cyano-2,3-dihydrophénalène

A un mélange fortement agité de 13,7 mmoles de 1,3-dihydrophénalèn-2-one et de 40 mmoles de cyanure de sodium dans 60 ml d'eau et 80 ml d'éther éthylique, est ajouté, goutte à goutte 1 ml d'acide chlorhydrique concentré. Après une heure d'agitation à 20°C, la phase organique est décantée, lavée par de l'eau, séchée et évaporée. Le résidu obtenu est traité par 20 ml d'une solution méthanolique d'ammoniac (3,5M), sous agitation pendant 5 heures à 20°C.
Après évaporation du solvant, l'huile résiduelle est reprise par 30 ml d'éther éthylique et extraite par de l'acide chlorhydrique 1N. Les phases acides réunies sont alcalinisées par de la soude à 35 % puis extraites par de l'éther. Après séchage et évaporation, on obtient le produit attendu sous forme d'huile.

### Stade B : 2-Amino-2-aminométhyl-2,3-dihydrophénalène

Le produit attendu est obtenu selon le procédé décrit au stade B de l'exemple 15.

### Stade C : Spiro[(1,3-diazacyclopent-1-ène)-5 : 2'-(2',3'-dihydrophénalène)], 1/2 fumarate

Le produit attendu est obtenu selon le procédé décrit au stade C de l'exemple 15.

Point de fusion : 245-250°C

| Microanalyse élémentaire : | | | |
|---|---|---|---|
| | C % | H % | N % |
| calculé | 72,84 | 5,75 | 9,99 |
| trouvé | 72,58 | 5,64 | 9,87 |

Les exemples 32 à 34 ont été obtenus selon le procédé décrit dans l'exemple 31.

### Exemple 32: Spiro[(1,3-diazacyclopent-1-ène)-5 : 2'-(5',8'-diméthyl-1',2',3',4'-tétrahydronaphtalène)], fumarate

Point de fusion : 192-194°C

| Microanalyse élémentaire : | | | |
|---|---|---|---|
| | C % | H % | N % |
| calculé | 65,44 | 6,71 | 8,48 |
| trouvé | 65,29 | 6,60 | 8,45 |

### Exemple 33: Spiro[(1,3-diazacyclopent-1-ène)-5 : 3'-(5',6'-benzochromane)], fumarate

Point de fusion : 200-204°C

| Microanalyse élémentaire : | | | |
|---|---|---|---|
| | C % | H % | N % |
| calculé | 64,40 | 5,12 | 7,91 |
| trouvé | 64,72 | 5,34 | 8,13 |

### Exemple 34: Spiro[(1,3-diazacyclopent-1-ène)-5 : 3'-(7',8'-benzochromane)], fumarate

Point de fusion : 185-190°C

| Microanalyse élémentaire : | | | |
|---|---|---|---|
| | C % | H % | N % |
| calculé | 64,40 | 5,12 | 7,91 |
| trouvé | 63,80 | 5,20 | 8,07 |

### Exemple 35: Spiro[(1-oxa-2-amino-3-aza-cyclopent-2-ène)-4 : 2'-(1',2',3',4'-tétrahydronaphtalène)], chlorhydrate

### Stade A : 2-Amino-2-cyano-1,2,3,4-tétrahydronaphtalène, chlorhydrate

34,2 mmoles de β-tétralone et 40 mmoles de cyanure de triméthylsilyle sont agitées à température ambiante. 25 mg d'iodure de zinc sont alors ajoutés au mélange précédent. Après 30 minutes d'agitation, 50 ml d'une solution méthanolique saturée en ammoniac sont ajoutés et l'ensemble est abandonné une nuit, sous agitation, à température ambiante.

Après évaporation du solvant, l'huile résiduelle est dissoute dans 500 ml d'éther, séchée puis additionnée de 5 ml d'éther chlorhydrique. Le produit attendu est obtenu par filtration du solide formé.

### Stade B : 2-Amino-2-aminocarbonyl-1,2,3,4-tétrahydronaphtalène, chlorhydrate

Une solution contenant 25,4 mmoles du composé obtenu au stade précédent dans 80 ml d'acide formique est refroidie à 0°C et saturée par de l'acide chlorhydrique anhydre.
A l'arrêt du dégagement gazeux, le solvant est évaporé et le résidu repris par 50 ml d'acétone. Le produit attendu est obtenu par filtration du solide blanc qui cristallise.

### Stade C : Acide 2-amino-1,2,3,4-tétrahydronaphtalène-2-carboxylique

Une suspension contenant 21,8 mmoles du composé obtenu au stade précédent dans 25 ml d'acide chlorhydrique 6N est portée à reflux jusqu'à dissolution complète. Le solvant est évaporé, le résidu repris par de l'isopropanol est à nouveau concentré. Le solide obtenu est dissous dans l'eau et le pH est amené à 7 par addition de soude 1N. Le produit attendu est le solide blanc formé qui est filtré et séché.

### Stade D : 2-Amino-2-hydroxyméthyl-1,2,3,4-tétrahydronaphtalène

A une solution contenant 20 mmoles du composé obtenu au stade précédent dans 50 ml de tétrahydrofurane anhydre, est ajoutée goutte à goutte sous atmosphère d'azote, à température ambiante, une suspension contenant 40 mmoles d'hydrure de lithium aluminium dans 100 ml de tétrahydrofurane. L'ensemble est porté une heure au reflux.
Après refroidissement à 0°C, 1,5 ml d'eau, 1,5 ml de soude 2,5N et 3 ml d'eau sont ajoutés successivement. L'ensemble est agité et le solide blanc qui se forme est filtré et lavé par du tétrahydrofurane. Les filtrats sont réunis, évaporés et conduisent au produit attendu sous forme d'huile.

### Stade E : Spiro[(1-oxa-2-amino-3-aza-cyclopent-2-ène)-4 : 2'-(1',2',3',4'-tétrahydronaphtalène)], chlorhydrate

Une solution contenant 12 mmoles de bromure de cyanogène dans 5 ml de dichlorométhane est additionnée rapidement, à 0 °C, à une solution contenant 10,6 mmoles du produit obtenu au stade précédent dans 20 ml de dichlorométhane.

L'ensemble est agité une nuit à température ambiante et le solide formé est filtré et lavé au dichlorométhane. Les filtrats réunis sont lavés par une solution de bicarbonate de potassium, séchés et évaporés.
Le produit attendu est obtenu après purification du résidu par chromatographie sur colonne de silice en utilisant comme éluant le dichlorométhane, un mélange dichlorométhane/éthanol (98/2) et enfin un mélange dichlorométhane/éthanol/ammoniaque (95/4,5/0,5). L'huile obtenue, dissoute dans de l'éther, est additionnée d'éther chlorhydrique. Le précipité est alors filtré et recristallisé dans un mélange isopropanol/éther.

Point de fusion : 206°C

| Microanalyse élémentaire : | | | | |
|---|---|---|---|---|
| | C % | H % | N % | Cl % |
| calculé | 60,38 | 6,33 | 11,73 | 14,85 |
| trouvé | 59,97 | 6,17 | 10,96 | 14,72 |

### Exemple 36: Spiro[(1,3-diaza-2-aminocyclopent-1-ène)-5 : 2'-(1',2',3',4'-tétrahydronaphtalène)], bromhydrate

A 5,7 mmoles de 2-amino-2-aminométhyl-1,2,3,4-tétrahydronaphtalène (obtenu au stade D de l'exemple 1) dissoutes dans 50 ml de dichlorométhane, sont ajoutées à 0 °C, 6,3 mmoles de bromure de cyanogène dans 5 ml de dichlorométhane. La solution est agitée une nuit à température ambiante. Le produit attendu qui se forme est filtré et recristallisé dans de l'isopropanol.

Point de fusion : 209°C

| Microanalyse élémentaire : | | | | |
|---|---|---|---|---|
| | C % | H % | N % | Br % |
| calculé | 51,08 | 5,72 | 14,89 | 28,32 |
| trouvé | 51,12 | 5,80 | 14,59 | 28,00 |

### Exemple 37: Spiro[(1-oxa-3-aza-cyclopent-2-ène)-4 : 2'-(1',2',3',4'-tétrahydronaphtalène)]

### Stade A : 2-Formamido-2-hydroxyméthyl-1,2,3,4-tétrahydronaphtalène

Une solution contenant 5,6 mmoles du composé obtenu au stade D de l'exemple 35 dans 5 ml de formiate d'éthyle est abandonnée une nuit à température ambiante. La solution est alors diluée par 25 ml de dichlorométhane, le solide, filtré, et le filtrat est évaporé. Le résidu est alors purifié par chromatographie sur colonne de silice en utilisant comme éluant un mélange dichlorométhane/éthanol (95/5).

### Stade B : Spiro[(1-oxa-3-aza-cyclopent-2-ène)4 : 2'-(1',2',3',4'-tétrahydronaphtalène)]

Une solution contenant 7,5 mmoles de diéthylaminosulfure trifluorure dans 3 ml de dichlorométhane est ajoutée, goutte à goutte, à - 10 °C sous atmosphère d'azote, à une solution contenant 3,5 mmoles du composé obtenu au stade précédent dans 40 ml de dichlorométhane.
Après 2 heures d'agitation à - 10 °C, 25 ml d'ammoniaque à 25 % sont ajoutés au mélange précédent. La phase organique est décantée, filtrée, séchée et évaporée. Le résidu solide est purifié sur colonne de silice en utilisant comme éluant un mélange dichlorométhane/éthanol (99/1). Le produit attendu est alors distillé à 125 °C sous pression réduite (p = 30 mmHg) puis recristallisé dans l'heptane à - 30 °C.

| Microanalyse élémentaire : | | | |
|---|---|---|---|
| | C % | H % | N % |
| calculé | 76,98 | 7,00 | 7,48 |
| trouvé | 76,83 | 7,42 | 7,58 |

Les exemples 38 à 43 ont été obtenus selon le procédé décrit dans l'exemple 1 en utilisant les produits de départ correspondants.

### Exemple 38: Spiro[(1,3-diazacyclopent-1-ène)-5 : 2'-(7'-isopropyl-1',2',3',4'-tétrahydronaphtalène)], 3/2 fumarate

Point de fusion : 192-194°C

| Microanalyse élémentaire : | | | |
|---|---|---|---|
| | C % | H % | N % |
| calculé | 62,67 | 6,51 | 6,96 |
| trouvé | 62,60 | 6,59 | 7,02 |

### Exemple 39: Spiro[(1,3-diazacyclopent-1-ène)-5 : 2'-(7'-tert-butyl-1',2',3',4'-tétrahydronaphtalène)], fumarate

Point de fusion : 188-190°C

| Microanalyse élémentaire : | | | |
|---|---|---|---|
| | C % | H % | N % |
| calculé | 67,02 | 7,31 | 7,82 |
| trouvé | 66,70 | 7,43 | 7,80 |

### Exemple 40: Spiro[(1,3-diazacyclopent-1-ène)-5 : 2'-(7'-éthyl-1',2',3',4'-tétrahydronaphtalène)], fumarate

Point de fusion : 148-152°C

| Microanalyse élémentaire : | | | |
|---|---|---|---|
| | C % | H % | N % |
| calculé | 65,44 | 6,71 | 8,48 |
| trouvé | 65,63 | 6,84 | 8,47 |

### Exemple 41: Spiro[(1,3-diazacyclopent-1-ène)-5 : 2'-(6',7'-diméthyl-1',2',3',4'-tétrahydronaphtalène)] , fumarate

Point de fusion : 189-192°C

| Microanalyse élémentaire : | | | |
|---|---|---|---|
| | C % | H % | N % |
| calculé | 65,44 | 6,71 | 8,48 |
| trouvé | 64,80 | 6,61 | 8,46 |

### Exemple 42: Spiro[(1,3-diazacyclopent-1-ène)-5 : 2'-(1',2',3',4',6',7',8'-heptahydrocyclopenta[g]naphtalène)], hémifumarate

de formule :

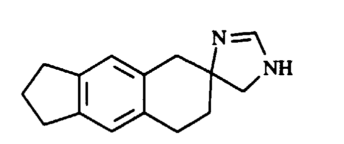

Point de fusion : 205-209°C

| Microanalyse élémentaire : | | | |
|---|---|---|---|
| | C % | H % | N % |
| calculé | 71,81 | 7,09 | 9,85 |
| trouvé | 71,40 | 6,99 | 9,49 |

### Exemple 43: Spiro[(1,3-diazacyclopent-1-ène)-5 : 2'-(5',6'-diméthylindane)], fumarate

Point de fusion : 214-216°C

| Microanalyse élémentaire : | | | |
|---|---|---|---|
| | C % | H % | N % |
| calculé | 64,54 | 6,37 | 8,85 |
| trouvé | 64,16 | 6,34 | 8,66 |

### Exemple 44: Spiro[(1,3-diazacyclopent-1-ène)-5 : 2'-(6'-isopropyloxy-1',2',3',4' tétrahydronaphtalène)], fumarate, isomère α

Le produit attenu est obtenu par dédoublement du composé de l'exemple 5 au moyen de l'acide D-di(paratoluyl)tartrique par recristallisations successives dans l'éthanol. La pureté énantiomérique est vérifiée par chromatographie chirale sur colonne DAICEL-OJ en utilisant comme éluant un mélange isopropanol/n-heptane/diéthylamine (10/90/0,1). Le sel est alors partagé entre la soude 1N et le dichlorométhane. Après extraction au dichlorométhane, les phases organiques sont séchées et évaporées. Le résidu est alors dissous dans de l'éthanol avec un équivalent d'acide fumarique et l'ensemble est porté à reflux. Le produit attendu est alors obtenu après refroidissement sous forme d'un solide blanc.

Point de fusion : 175-177°C

| Microanalyse élémentaire : | | | |
|---|---|---|---|
| | C % | H % | N % |
| calculé | 63,32 | 6,71 | 7,77 |
| trouvé | 62,91 | 6,59 | 7,73 |

### Exemple 45: Spiro[(1,3-diazacyclopent-1-ène)-5 : 2'-(6'-isopropyloxy-1',2',3',4'-tétrahydronaphtalène)], fumarate, isomère β

Le produit attendu est obtenu selon le procédé décrit dans l'exemple 44, en utilisant pour le dédoublement l'acide L-di(para-toluyl)tartrique.

Point de fusion : 175-177°C

| Microanalyse élémentaire : | | | |
|---|---|---|---|
| | C % | H % | N % |
| calculé | 63,32 | 6,71 | 7,77 |
| trouvé | 63,08 | 6,77 | 7,67 |

### Exemple 46: (S)-Spiro[(1,3-diazacyclopent-1-ène)-5 : 2'-(7'-méthyl-1',2',3',4'-tétrahydronaphtalène)], fumarate

### Stade A : (S)-2-[(R)-α-Méthylbenzylamino]-2-cyano-1,2,3,4-tétrahydronaphtalène

A une solution maintenue sous azote, de 20 mmoles de 7-méthyl-2-tétralone dans un mélange contenant 13 ml d'éthanol, 3,2 ml de méthanol et 3 ml d'eau sont ajoutées successivement 13 mmoles de chlorhydrate de (R)-(+)-α-méthylbenzylamine (de pureté énantiomérique supérieure à 99 %) puis 19 mmoles de cyanure de sodium. Le mélange est agité 20 heures à 20°C. Le précipité formé est alors filtré, lavé par de l'éthanol glacé et séché et conduit au produit attendu.

### Stade B : (S)-2-[(R)-α-Méthylbenzylamino]-2-aminométhyl-1,2,3,4-tétrahydronaphtalène

A une suspension contenant 35 mmoles d'hydrure de lithium aluminium dans 60 ml d'éther anhydre, est additionnée goutte à goutte une solution contenant 15 mmoles du composé obtenu au stade précédent dans 30 ml d'éther, en maintenant la température à 30°C. Après 2 heures d'agitation, le mélange est refroidi à 5°C et hydrolysé par 1,3 ml d'eau, 1,50 ml de soude 2N et 2,5 ml d'eau. Après filtration, le filtrat est évaporé. Le résidu est dissous dans 50 ml d'acétate d'éthyle. Cette phase est lavée à l'eau puis extraite par de l'acide chlorhydrique 1N. Les phases aqueuses acides sont alcalinisées par de la soude à 35 % et extraites par de l'acétate d'éthyle. Le produit attendu est obtenu après séchage et évaporation des phases organiques.

### Stade C : (S)-2-Amino-2-aminométhyl-1,2,3,4-tétrahydronaphtalène

Une solution contenant 10 mmoles du composé obtenu au stade précédent dans 40 ml de méthanol et 3,5 ml d'acide acétique est hydrogénée à pression atmosphérique en présence de 500 mg de dihydroxyde de palladium pendant 20 heures. Après filtration du catalyseur, le filtrat est évaporé et le résidu est dissous dans 40 ml d'acétate d'éthyle. Cette phase est lavée à l'eau et extraite par de l'acide chlorhydrique 1N. Les phases acides sont alcalinisées par de la soude à 35 % et extraites par du dichlorométhane. Les phases organiques sont séchées et évaporées et conduisent au produit attendu sous forme d'huile incolore.

### Stade D : (S)-Spiro[(1,3-diazacyclopent-1-ène)-5: 2'-(7'-méthyl-1',2',3',4'-tétrahydronaphtalène)], fumarate

Un mélange contenant 8 mmoles du composé obtenu au stade précédent et 8 mmoles d'acétate de formamidine dans 30 ml d'éthanol est agité, à 20°C, sous atmosphère d'azote, pendant 12 heures. Le solvant est évaporé et le résidu est repris par 20 ml d'acide chlorhydrique 1N. Cette phase est lavée par de l'acétate d'éthyle puis alcalinisée par de la soude à 35 %. Après extraction par de l'acétate d'éthyle, les phases organiques sont lavées par une solution aqueuse de chlorure de sodium puis évaporées.Le résidu est alors dissous dans 15 ml d'éthanol puis traité par un équivalent d'acide fumarique dans 10 ml d'éthanol. Le produit attendu est alors obtenu après évaporation du solvant et cristallisation dans un mélange éthanol/éther. La pureté énantiomérique est vérifiée par chromatographie chirale sur colonne DIACEL-OJ en utilisant comme éluant un mélange isopropanol/n-heptane/diéthylamine (60/1000/0,8). La configuration absolue a été déterminée par diffraction de Rayons X sur un monocristal du produit.

Point de fusion : 162-164°C

| Microanalyse élémentaire : | | | |
|---|---|---|---|
| | C % | H % | N % |
| calculé | 64,54 | 6,37 | 8,85 |
| trouvé | 64,19 | 6,29 | 8,87 |

### Exemple 47: (R)-Spiro[(1,3-diazacyclopent-1-ène)-5 : 2'-(7'-méthyl-1',2',3',4'-tétrahydronaphtalène)], fumarate

Le produit attendu est obtenu selon le procédé décrit dans l'exemple 46, en utilisant au stade A la (S)-(-)-α-méthylbenzylamine.

Point de fusion : 162-164°C

| Microanalyse élémentaire : | | | |
|---|---|---|---|
| | C % | H % | N % |
| calculé | 64,54 | 6,37 | 8,85 |
| trouvé | 64,54 | 6,33 | 8,79 |

### Etude pharmacologique des composés de l'invention

### Exemple 48: Etude in vitro sur les artères fémorales et les veines saphènes de chien

La technique utilisée s'inspire de celle décrite par FOWLER et Coll. (J. Pharmacol. Exp. Ther., 229, 712-718, 1984). Des chiens bâtards mâles ou femelles d'environ 15-25 kg ont été utilisés comme source d'organes. Les animaux sont anesthésiés au pentobarbital (30 mg/kg, en intraveineux). Les pattes sont incisées et les vaisseaux prélevés. Ils sont placés dans du liquide de Krebs-Ringer (NaCl 118 mM ; NaHCO₃ 25 mM ; Glucose 10 mM ; KCl 4,7 mM; CaCl₂ 1,25 mM ; MgSO₄ 1,19 mM ; KH₂PO₄ 1,14 mM) à température ambiante et sous bullage de carbogène (95 % O₂, 5 % CO₂). Ces vaisseaux sont alors soigneusement débarrassés de leur graisse puis découpés en anneaux de 2 mm de large et montés sous une tension de base de 4 g (artères fémorales) ou 1 g (veines saphènes) dans des cuves thermostatées à 37°C contenant du liquide de Krebs-Ringer constamment bullé par du carbogène. Un crochet inférieur constitue le point fixe, tandis que le crochet supérieur est relié à un capteur de force isométrique. Les variations de tension sont digitalisées, stockées sur disque et traitées par un système informatique. Après montage, les organes sont laissés en repos pendant 90 minutes, des rinçages étant effectués toutes les 30 min. Après réajustement de la tension de base, une contraction est provoquée par une dose unique de KCl (100 mM). Après stabilisation, lavages et retour à la ligne de base, une contraction est provoquée par une dose unique de phényléphrine (concentration submaximale) afin de régulariser les contractions suivantes. Après lavage et retour à la ligne de base, une courbe effet/concentration est réalisée par une adjonction de doses cumulatives d'agoniste (l'espacement entre les doses est d'un demi-log). Cette expérience permet de calculer la concentration efficace 50 % (EC₅₀) de la manière suivante : les valeurs de tension sont d'abord converties en pourcentages par rapport à l'effet maximum provoqué par 100 mM de KCl. Cette EC₅₀ est déterminée par régression non linéaire par la méthode SIMPLEX (M.S. CACECI, Byte, 340-362, 1984), calculée suivant le modèle de la loi d'action de masse de L. MICHAELIS et M.L. MENTEN (Biochem. Zeitschrift, 49, 333-369, 1913).

E = (Emax*Cⁿ)(ECⁿ+Cⁿ) avec E = effet ; Emax = effet maximum ;
C = concentration ; EC = EC₅₀ ; n = nombre de HILL

Les produits de l'invention contractent les artères et les veines de chien. Le maximum de ces contractions est plus faible que celui obtenu avec le KCl. Les résultats obtenus sont présentés dans le tableau ci-dessous :

| Exemple | ARTERE | | VEINE | |
|---|---|---|---|---|
| | EC₅₀ (µM) | Max (% KCl) | EC₅₀ (µM) | Max (% KCl) |
| 1 | 5,9 | 19 | 1,3 | 98 |
| 8 | 13 | 12 | 2,1 | 44 |
| 9 | 0,8 | 60 | 3,9 | 81 |
| 17 | 24 | 10 | 1,9 | 44 |
| 24 | 70 | 8 | 0,16 | 57 |
| 31 | 0,5 | 10 | 1,6 | 44 |
| 46 | 15 | 36 | 1,86 | 48 |

### Exemple 49: Etude in vivo chez le RAT AMYELE

Des rats Sprague Dawley mâles (300-400 g) sont anesthésiés à l'éther. La trachée est canulée, la moëlle épinière est détruite au moyen d'une tige en acier et l'animal est immédiatement mis sous respiration artificielle. Les nerfs vagues sont sectionnés. Les artères carotides sont ligaturées, un cathéter est placé dans l'une et sert à enregistrer la pression artérielle. Trois autres cathéters sont placés dans les veines jugulaires et la veine du pénis et servent aux injections. La température des animaux est maintenue à 36°C. L'animal est prétraité par une injection de tertatolol (100 µg/kg). L'animal est également prétraité 10 minutes après par le prazosin (100 µg/kg) ou la yohimbine (1 mg/kg) lorsque l'on veut déterminer les propriétés alpha₁ ou alpha₂-adrénergiques du produit. Dix minutes plus tard, des doses cumulatives croissantes de produit sont injectées toutes les 20 secondes. Les variations de pression artérielles sont détectées à l'aide d'une cellule de pression P23XL Statham et sont enregistrées. Les valeurs de pression sont exprimées en mm Hg. Cette expérience permet de calculer la concentration augmentant la pression de 20 mm Hg (C₂₀) par régression non linéaire suivant le modèle de la loi d'action de masse de Michaelis et Menten comme décrit ci-dessus. L'effet maximum obtenu est ensuite converti en pourcentage par rapport à l'effet maximum provoqué par la phényléphrine. Les composantes alpha₁ ou alpha₂-adrénergiques du produit sont apppréciées à l'aide du rapport des C₂₀ obtenues en présence de prazosin ou de yohimbine sur les valeurs obtenues en absence de ces antagonistes. Chez le rat amyélé, les produits de l'invention produisent des hypertensions sensibles au prazosin et à la yohimbine. Les résultats sont rassemblés dans le tableau ci-dessous :

| Exemple | C₂₀ (µg/kg) | Ratio C₂₀ traité / C₂₀ contrôle | |
|---|---|---|---|
| | Contrôle | Prazosin | Yohimbine |
| 1 | 4 | 1,9 | 4,7 |
| 3 | 0,9 | 9,2 | 2,8 |
| 5 | 68 | 11 | 66 |
| 7 | 137 | 2,9 | 2,4 |
| 24 | 30 | 1,4 | 1,5 |
| 46 | 6,8 | 3,7 | 3,4 |

### Exemple 50: Etude in vivo du débit carotidien chez le chien anesthésié

Les chiens (12-18 kg) sont anesthésiés au pentobarbital (30 mg/kg i.v.). L'anesthésie est ensuite maintenue par une perfusion de 0,8 ml/h de pentobarbital 6 %. Les animaux sont placés sous respiration assistée et leur température est maintenue à 37°C (couverture thermostatée Harvard). La pression artérielle et la fréquence cardiaque sont mesurées grâce à une sonde Millar dans l'artère fémorale ou l'artère brachiale, par l'intermédiaire d'un transducer et d'un biotach Gould. Le débit sanguin des artères carotides est enregistré grâce à un débimètre électromagnétique Gould SP2202.
Dans ce modèle, le produit anti-migraineux, le Sumatriptan à 300 µg/kg i.v. provoque une baisse du débit carotidien de 47 ± 6 % (n = 5). Le composé de l'exemple 46 de la présente invention, à la dose de 5 µg/kg i.v., provoque une baisse du débit carotidien de 62 ± 4 % (n = 6).

### Exemple 51: Composition pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 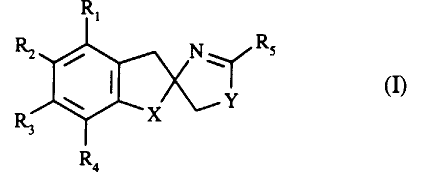 dans laquelle :
- X représente -CH₂-, -(CH₂)₂-, -CH=CH-, -O-CH₂-, -S-CH₂-, -SO-CH₂- ou -SO₂-CH₂-,
- Y représente un atome d'oxygène, de soufre, ou un groupement -NR₆-,
- R₁ représente un atome d'hydrogène ou un groupement alkyle (C₁-C₆) linéaire ou ramifié,
- R₂ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₃ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₄ représente un atome d'hydrogène (à la condition que dans ce cas R₁ représente un atome d'hydrogène), un atome d'halogène, un groupement alkyle (C₁-C₆) linéaire ou ramifié (substitué ou non par un ou plusieurs atomes d'halogène) ou un groupement hydroxy,
ou bien
- R₁ et R₂, R₂ et R₃, R₃ et R₄ ou R₄ et X forment ensemble avec les atomes de carbone qui les portent, un cycle benzénique à la condition que, dans le cas où R₁ et R₂ forment un cycle benzénique X soit différent de -CH₂- ou -(CH₂)₂-,
- R₅ représente un atome d'hydrogène ou un groupement amino (substitué ou non par un ou deux groupements alkyle (C₁-C₆) linéaire ou ramifié),
- R₆ a la même signification que R₁,
leurs isomères ainsi que leurs sels d'addition à un acide pharmaceutiquement acceptable.

2. Composés de formule (I) selon la revendication 1 tels que X représente -(CH₂)₂-, leurs isomères ainsi que leurs sels d'addition à un acide pharmaceutiquement acceptable.

3. Composés de formule (I) selon la revendication 1 tels que Y représente -NH-, leurs isomères ainsi que leurs sels d'addition à un acide pharmaceutiquement acceptable.

4. Composés de formule (I) selon la revendication 1 tels que R₁ représente un atome d'hydrogène, leurs isomères ainsi que leurs sels d'addition à un acide pharmaceutiquement acceptable.

5. Composés de formule (I) selon la revendication 1 tels que R₅ représente un atome d'hydrogène, leurs isomères ainsi que leurs sels d'addition à un acide pharmaceutiquement acceptable.

6. Composé de formule (I) selon la revendication 1 qui est le spiro[(1,3-diazacyclopent-1-ène)-5 : 2'-(1',2',3',4'-tétrahydronaphtalène)], ses isomères ainsi que ses sels d'addition à un acide pharmaceutiquement acceptable.

7. Composé de formule (I) selon la revendication 1 qui est le spiro[(1,3-diazacyclopent-1-ène)-5 : 2'-(7'-méthyl-1',2',3',4'-tétrahydronaphtalène)], ses isomères ainsi que ses sels d'addition à un acide pharmaceutiquement acceptable.

8. Procédé de préparation des composés de formule (I) selon la revendication 1 pour lesquels Y représente un groupement -NR₆-, caractérisé en ce que l'on utilise comme produit de départ un composé de formule (II) : 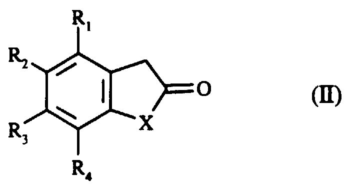 dans laquelle X, R₁, R₂, R₃ et R₄ ont la même signification que dans la formule (I)
que l'on fait réagir :
- soit avec de la benzylamine en présence d'acide paratoluènesulfonique pour conduire au composé de formule (III) : 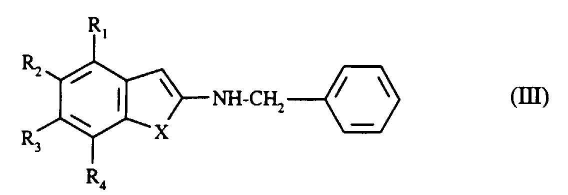 dans laquelle X, R₁, R₂, R₃ et R₄ ont la même signification que dans la formule (I)
que l'on fait réagir, sous atmosphère inerte, avec du cyanure de triméthylsilyle en présence d'iodure de zinc,
pour conduire au composé de formule (IV) : 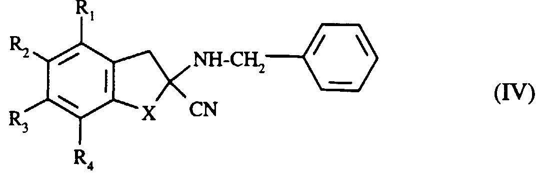 dans laquelle X, R₁, R₂, R₃ et R₄ ont la même signification que dans la formule (I),
que l'on réduit à l'aide d'hydrure de lithium aluminium, puis par hydrogénation catalytique pour conduire au composé de formule (V) : 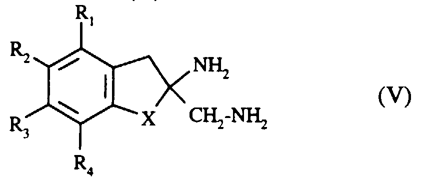 dans laquelle X, R₁, R₂, R₃ et R₄ ont la même signification que dans la formule (I),
- soit avec du cyanure de potassium en présence de chlorure d'ammonium en milieu inerte avec du cyanure de sodium en milieu acide, ou bien avec du cyanure de triméthylsilyle présence d'iodure de zinc puis avec une solution alcoolique saturée en ammoniac, pour conduire au composé de formule (VI) : 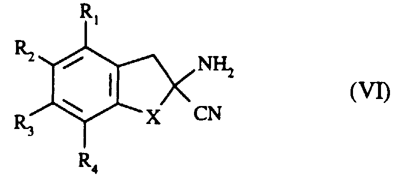 dans laquelle X, R₁, R₂, R₃ et R₄ ont la même signification que dans la formule (I),
que l'on réduit à l'aide d'hydrure de lithium aluminium pour conduire au composé de formule (V) décrit précédemment,
composé de formule (V),
que l'on fait réagir avec de la formamidine en milieu alcoolique, un formiate d'alkyle ou avec un halogènure de cyanogène (suivie, selon la nature du composé de formule (I) que l'on souhaite obtenir, d'une réaction d'alkylation à l'aide d'un halogénure d'alkyle),
pour conduire au composé de formule (I/a), cas particulier des composés de formule (I) : 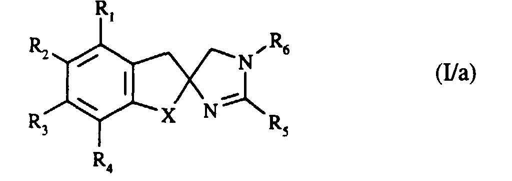 dans laquelle X, R₁, R₂, R₃, R₄, R₅ et R₆ ont la même signification que dans la formule (I)
composé de formule (I/a),
que l'on purifie, le cas échéant, selon une technique classique de purification,
dont on sépare, si on le souhaite, les isomères selon une technique classique de purification,
et que l'on transforme, éventuellement, en ses sels d'addition à un acide pharmaceutiquement acceptable.

9. Procédé de préparation des composés de formule (I) selon la revendication 1 pour lesquels Y représente un atome d'oxygène ou de soufre caractérisé en ce que l'on fait réagir avec de l'acide formique en milieu anhydre saturé en acide chlorhydrique le composé de formule (VI) selon la revendication 7,
pour conduire au composé de formule (VII) : 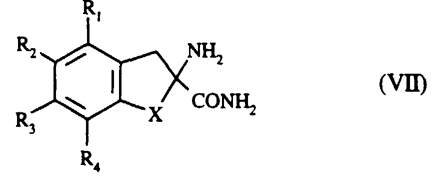 dans laquelle R₁, R₂, R₃, R₄ et X ont la même signification que dans la formule (I),
que l'on transforme en acide correspondant de formule (VIII) en milieu chlorhydrique concentré : 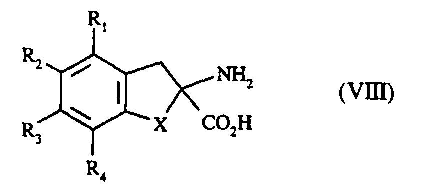 dans laquelle R₁, R₂, R₃, R₄ et X ont la même signification que dans la formule (I),
qui subit une réduction par l'hydrure de lithium aluminium en milieu inerte,
pour conduire au composé de formule (IXa) : 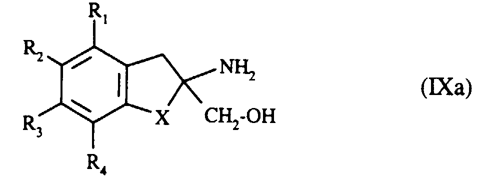 dans laquelle R₁, R₂, R₃, R₄ et X ont la même signification que dans la formule (I),
composé de formule (IXa), que l'on transforme, selon la nature des composés de formule (I) que l'on souhaite obtenir, en tosylate correspondant à l'aide d'acide p.toluènesulfonique puis que l'on fait réagir avec de la thiourée ou de l'acide thioacétique,
pour conduire, après hydrolyse, au composé de formule (IXb) : 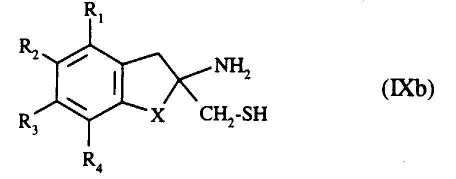 dans laquelle R₁, R₂, R₃, R₄ et X ont la même signification que dans la formule (I),
composé de formule (IXa) ou (IXb) que l'on fait réagir avec de la formamidine en milieu alcoolique, un formiate d'alkyle ou avec un halogénure de cyanogène (suivie, selon la nature du composé de formule (I) que l'on souhaite obtenir, d'une réaction d'alkylation à l'aide d'un halogénure d'alkyle),
pour conduire au composé de formule (I/b), cas particulier des composés de formule (I) : 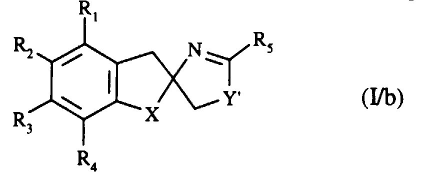 dans laquelle R₁, R₂, R₃, R₄, R₅ et X ont la même signification que précédemment et Y' représente un atome d'oxygène ou de soufre,
composé de formule (I/b)
que l'on purifie, le cas échéant, selon une technique classique de purification,
dont on sépare, si on le souhaite, les isomères selon une technique classique de purification,
et que l'on transforme, éventuellement, en ses sels d'addition à un acide pharmaceutiquement acceptable.

10. Compositions pharmaceutiques contenant comme principe actif au moins un composé selon l'une quelconque des revendications 1 à 7 seul ou en combinaison avec un ou plusieurs excipients ou véhicules inertes, non toxiques, pharmaceutiquement acceptables.

11. Compositions pharmaceutiques selon la revendication 9 contenant au moins un principe actif selon l'une quelconque des revendications 1 à 7 utilisé en tant qu'agoniste partiel α₁ et α₂ adrénergique dans le traitement de la maladie veineuse et de la migraine.

## Patentansprüche

1. Verbindungen der Formel (I): 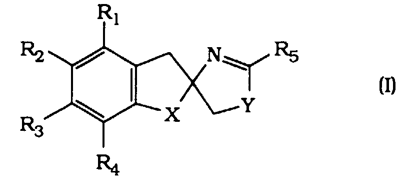 in der:
- X -CH₂-, -(CH₂)₂-, -CH=CH-, -O-CH₂-, -S-CH₂-, -SO-CH₂- oder -SO₂-CH₂-,
- Y ein Sauerstoffatom, ein Schwefelatom oder eine Gruppe -NR₆-,
- R₁ ein Wasserstoffatom oder eine geradkettige oder verzweigte (C₁-C₆)-Alkylgruppe,
- R₂ ein Wasserstoffatom, ein Halogenatom, eine geradkettige oder verzweigte (gegebenenfalls durch ein oder mehrere Halogenatome substituierte) (C₁-C₆)-Alkylgruppe, eine Hydroxygruppe, eine geradkettige oder verzweigte (C₁-C₆)-Alkoxygruppe oder eine geradkettige oder verzweigte (C₁-C₆)-Alkylthiogruppe,
- R₃ ein Wasserstoffatom, ein Halogenatom, eine geradkettige oder verzweigte (gegebenenfalls durch eine oder mehrere Halogenatome substituierte) (C₁-C₆)-Alkylgruppe, eine Hydroxygruppe, eine geradkettige oder verzweigte (C₁-C₆)-Alkoxygruppe oder eine geradkettige oder verzweigte (C₁-C₆)-Alkylthiogruppe,
- R₄ ein Wasserstoffatom (mit der Maßgabe, daß in diesem Fall R₁ ein Wasserstoffatom darstellt), ein Halogenatom, eine geradkettige oder verzweigte (gegebenenfalls durch ein oder mehrere Halogenatome substituierte) (C₁-C₆)-Alkylgruppe oder eine Hydroxygruppe,
oder
- R₁ und R₂, R₂ und R₃, R₃ und R₄ oder R₄ und Xgemeinsam mit den sie tragenden Kohlenstoffatomen einen Benzolring, mit der Maßgabe, daß dann, wenn R₁ und R₂ einen Benzolring bilden, X von -CH₂- oder -(CH₂)₂- verschieden ist,
- R₅ ein Wasserstoffatom oder eine (gegebenenfalls durch eine oder zwei geradkettige oder verzweigte (C₁-C₆)-Alkylgruppen substituierte) Aminogruppe bedeuten und
R₆ die gleichen Bedeutungen besitzt wie R₁,
deren Isomere sowie deren Additionssalze mit einer pharmazeutisch annehmbaren Säure.

2. Verbindungen der Formel (I) nach Anspruch 1, in der X -(CH₂)₂- darstellt, deren Isomere sowie deren Additionssalze mit einer pharmazeutisch annehmbaren Säure.

3. Verbindungen der Formel (I) nach Anspruch 1, in der Y -NH- darstellt, deren Isomere sowie deren Additionssalze mit einer pharmazeutisch annehmbaren Säure.

4. Verbindungen der Formel (I) nach Anspruch 1, in der R₁ ein Wasserstoffatom darstellt, deren Isomere sowie deren Additionssalze mit einer pharmazeutisch annehmbaren Säure.

5. Verbindungen der Formel (I) nach Anspruch 1, in der R₅ ein Wasserstoffatom darstellt, deren Isomere sowie deren Additionssalze mit einer pharmazeutisch annehmbaren Säure.

6. Verbindung der Formel (I) nach Anspruch 1, nämlich Spiro[5-(1,3-diazacyclopent-1-en): 2'-(1',2',3',4'-tetrahydronaphthalin)], dessen Isomere und dessen Additionssalze mit einer pharmazeutisch annehmbaren Säure.

7. Verbindung der Formel (I) nach Anspruch 1, nämlich Spiro[5-(1,3-diazacyclopent-1-en) : 2'-(7'-methyl-1',2',3',4'-tetrahydronaphthalin)], dessen Isomere und dessen Additionssalze mit einer pharmazeutisch annehmbaren Säure.

8. Verfahren zur Herstellung der Verbindungen der Formel (I) nach Anspruch 1, in der Y eine Gruppe -NR₆- darstellt, dadurch gekennzeichnet, daß man als Ausgangsprodukt eine Verbindung der Formel (II) verwendet: 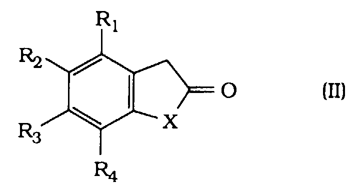 in der X, R₁, R₂, R₃ und R₄ die bezüglich der Formel (I) angegebenen Bedeutungen besitzen,
welche man:
- entweder mit Benzylamin in Gegenwart von p-Toluolsulfonsäure umsetzt zur Bildung der Verbindung der Formel (III): 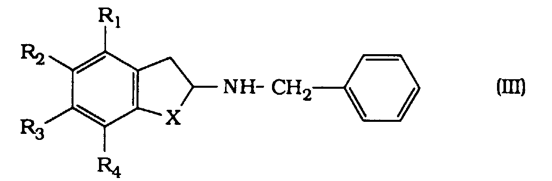 in der X, R₁, R₂, R₃ und R₄ die bezüglich der Formel (I) angegebenen Bedeutungen besitzen,
welche man unter einer inerten Atmosphäre in Gegenwart von Zinkiodid mit Trimethylsilylcyanid umsetzt zur Bildung der Verbindung der Formel (IV): 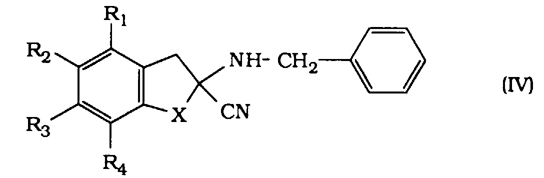 in der X, R₁, R₂, R₃ und R₄ die bezüglich der Formel (I) angegebenen Bedeutungen besitzen,
welche man mit Lithiumaluminiumhydrid und dann durch katalytische Hydrierung reduziert zur Bildung der Verbindung der Formel (V): 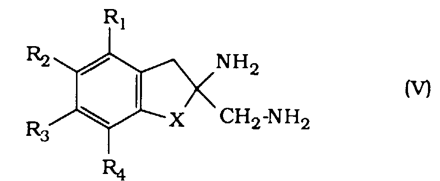 in der X, R₁, R₂, R₃ und R₄ die bezüglich der Formel (I) angegebenen Bedeutungen besitzen,
- oder mit Kaliumcyanid in Gegenwart von Ammoniumchlorid in einem inerten Medium oder mit Natriumnitrid in saurem Medium oder mit Trimethylsilylcyanid in Gegenwart von Zinkiodid und dann mit einer mit Ammoniak gesättigten alkoholischen Lösung behandelt zur Bildung der Verbindung der Formel (VI): 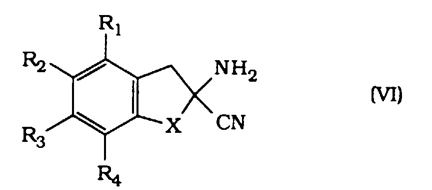 in der X, R₁, R₂, R₃ und R₄ die bezüglich der Formel (I) angegebenen Bedeutungen besitzen,
welche man mit Lithiumaluminiumhydrid reduziert zur Bildung der Verbindung der oben definierten Formel (V),
welche Verbindung der Formel (V)
man mit Formamidin in alkoholischem Medium, einem Alkylformiat oder mit einem Cyanogenhalogenid umsetzt (gefolgt in Abhängigkeit von der Art der herzustellenden Verbindung der Formel (I) von einer Alkylierungsreaktion mit Hilfe eines Alkylhalogenids)
zur Bildung der Verbindung der Formel (I/a), einem Sonderfall der Verbindungen der Formel (I): 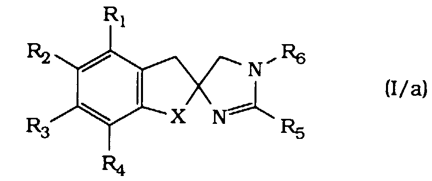 in der X, R₁, R₂, R₃, R₄, R₅ und R₆ die bezüglich der Formel (I) angegebenen Bedeutungen besitzen,
welche Verbindung der Formel (I/a)
man gegebenenfalls mit Hilfe einer klassischen Reinigungsmethode reinigt, gewünschtenfalls mit Hilfe einer klassischen Reinigungsmethode in die Isomeren auftrennt
und gegebenenfalls mit Hilfe einer pharmazeutisch annehmbaren Säure in ihre Additionssalze überführt.

9. Verfahren zur Herstellung der Verbindungen der Formel (I) nach Anspruch 1, in der Y ein Sauerstoffatom oder ein Schwefelatom darstellt, dadurch gekennzeichnet, daß man die Verbindung der Formel (VI) nach Anspruch 8 in einem wasserfreiem, mit Chlorwasserstoffsäure gesättigten Medium mit Ameisensäure umsetzt
zur Bildung der Verbindung der Formel (VII): 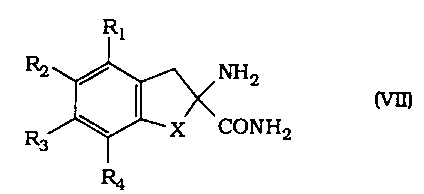 in der R₁, R₂, R₃, R₄ und X die bezüglich der Formel (I) angegebenen Bedeutungen besitzen,
welche man in konzentriertem, chlorwasserstoffsaurem Medium in die entsprechende Säure der Formel (VIII) umwandelt: 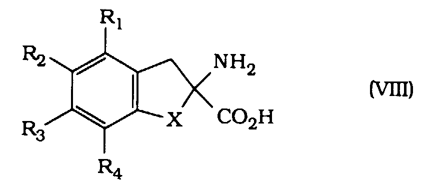 in der R₁, R₂, R₃, R₄ und X die bezüglich der Formel (I) angegebenen Bedeutungen besitzen,
welche man einer Reduktion mit Lithiumaluminiumhydrid in inertem Medium unterwirft zur Bildung der Verbindung der Formel (IXa): 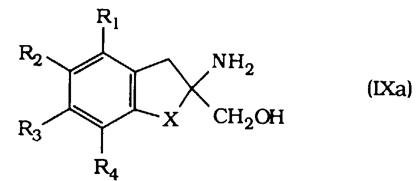 in der R₁, R₂, R₃, R₄ und X die bezüglich der Formel (I) angegebenen Bedeutungen besitzen,
welche Verbindung der Formel (IXa) man in Abhängigkeit von der Art der herzustellenden Verbindungen der Formel (I) mit p-Toluolsulfonsäure in das entsprechende Tosylat und dann mit Thioharnstoff oder Thioessigsäure umsetzt, so daß man nach der Hydrolyse die Verbindung der Formel (IXb) erhält: 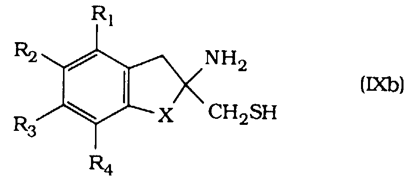 in der R₁, R₂, R₃, R₄ und X die bezüglich der Formel (I) angegebenen Bedeutungen besitzen,
welche Verbindung der Formel (IXa) oder (IXb) man mit Formamidin in alkoholischem Medium, mit einem Alkylformiat oder mit einem Cyanogenhalogenid umsetzt (gefolgt in Abhängigkeit von der Art der herzustellenden Verbindung der Formel (I) von einer Alkylierungsreaktion mit Hilfe eines Alkylhalogenids), zur Bildung der Verbindung der Formel (I/b), einem Sonderfall der Verbindungen der Formel (I): 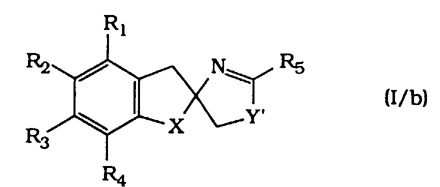 in der R₁, R₂, R₃, R₄, R₅ und X die oben angegebenen Bedeutungen besitzen und Y' ein Sauerstoffatom oder ein Schwefelatom darstellt,
welche Verbindung der Formel (I/b) man
gegebenenfalls mit Hilfe einer klassischen Reinigungsmethode reinigt, gewünschtenfalls mit Hilfe einer klassischen Reinigungsmethode in die Isomeren auftrennt und
gegebenenfalls mit Hilfe einer pharmazeutisch annehmbaren Säure in ihre Additionssalze überführt.

10. Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien oder Bindemitteln.

11. Pharmazeutische Zubereitungen nach Anspruch 10 enthaltend mindestens einen Wirkstoff nach einem der Ansprüche 1 bis 7 zur Verwendung als teilweiser α₁ - und α₂-adrenergischer Agonist bei der Behandlung von Venenerkrankungen und der Migräne.

## Claims

1. Compounds of formula (I): 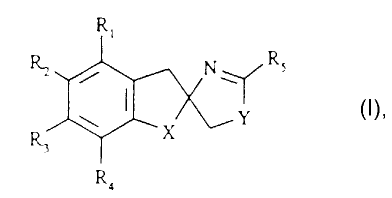 wherein:
- X represents -CH₂-, -(CH₂)₂-, -CH=CH-, -O-CH₂-, -S-CH₂-, -SO-CH₂ or SO₂-CH₂-,
- Y represents an oxygen or sulphur atom or a group -NR₆-,
- R₁ represents a hydrogen atom or a linear or branched (C₁-C₆)alkyl group,
- R₂ represents a hydrogen or halogen atom, a linear or branched (C₁-C₆)alkyl group (unsubstituted or substituted by one or more halogen atoms), a hydroxy group, a linear or branched (C₁-C₆)alkoxy group or a linear or branched (C₁-C₆)alkylthio group,
- R₃ represents a hydrogen or halogen atom, a linear or branched (C₁-C₆)alkyl group (unsubstituted or substituted by one or more halogen atoms), a hydroxy group, a linear or branched (C₁-C₆)alkoxy group or a linear or branched (C₁-C₆)alkylthio group,
- R₄ represents a hydrogen atom (with the proviso that, in that case, R₁ represents a hydrogen atom), a halogen atom, a linear or branched (C₁-C₆)alkyl group (unsubstituted or substituted by one or more halogen atoms) or a hydroxy group,
or
- R₁ and R₂, R₂ and R₃, R₃ and R₄, or R₄ and X, together with the carbon atoms carrying them, form a benzene ring, with the proviso that, when R₁ and R₂ form a benzene ring, X is other than -CH₂- or -(CH₂)₂-,
- R5 represents a hydrogen atom or an amino group (unsubstituted or substituted by one or two linear or branched (C₁-C₆)alkyl groups),
- R₆ is as defined for R₁,
their isomers, and addition salts thereof with a pharmaceutically acceptable acid.

2. Compounds of formula (I) according to claim 1, wherein X represents -(CH₂)₂-, their isomers, and addition salts thereof with a pharmaceutically acceptable acid.

3. Compounds of formula (I) according to claim 1, wherein Y represents -NH-, their isomers, and addition salts thereof with a pharmaceutically acceptable acid.

4. The compounds of formula (I) according to claim 1, wherein R₁ represents a hydrogen atom, their isomers, and addition salts thereof with a pharmaceutically acceptable acid.

5. The compounds of formula (I) according to claim 1, wherein R₅ represents a hydrogen atom, their isomers, and addition salts thereof with a pharmaceutically acceptable acid.

6. Compound of formula (I) according to claim 1 that is spiro[(1,3-diazacyclopent-1-ene)-5 : 2'-(1',2',3',4'-tetrahydronaphthalene)], its isomers and addition salts thereof with a pharmaceutically acceptable acid.

7. Compound of formula (I) according to claim 1 that is spiro[(1,3-diazacyclopent-1-ene)-5 : 2'-(7'-methyl-1',2',3',4'-tetrahydronaphthalene)], its isomers and addition salts thereof with a pharmaceutically acceptable acid.

8. Process for the preparation of compounds of formula (I) according to claim 1 wherein Y represents a group -NR₆-, characterised in that there is used as starting material a compound of formula (II): 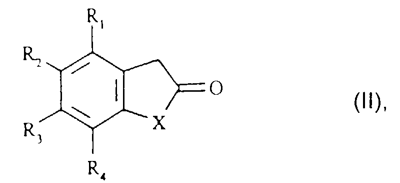 wherein X, R₁, R₂, R₃ and R₄ are as defined for formula (I),
which is reacted :
- either with benzylamine in the presence of para-toluenesulphonic acid to yield a compound of formula (III): 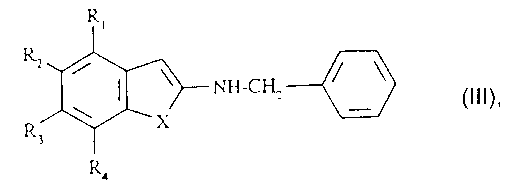 wherein X, R₁, R₂, R₃ and R₄ are as defined for formula (I),
which is reacted, under an inert atmosphere, with trimethylsilyl cyanide in the presence of zinc iodide
to yield a compound of formula (IV): 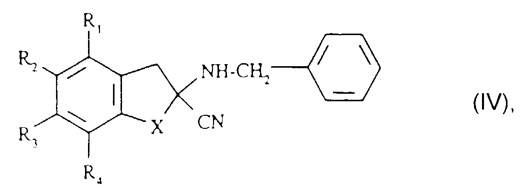 wherein X, R₁, R₂, R₃ and R₄ are as defined for formula (I),
which is reduced with lithium aluminium hydride and then by catalytic hydrogenation to yield a compound of formula (V): 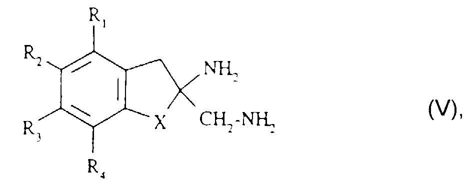 wherein X, R₁, R₂, R₃ and R₄ are as defined for formula (I),
- or with potassium cyanide in the presence of ammonium chloride in an inert medium or with sodium cyanide in an acid medium, or with trimethylsilyl cyanide in the presence of zinc iodide and then with an alcoholic saturated ammonia solution, to yield a compound of formula (VI): 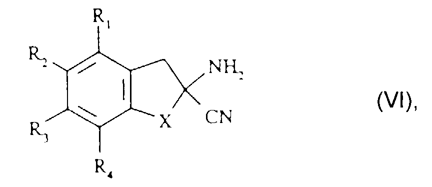 wherein X, R₁, R₂, R₃ and R₄ are as defined for formula (I),
which is reduced with lithium aluminium hydride to yield a compound of formula (V) described previously,
which compound of formula (V) is reacted with formamidine in an alcoholic medium, an alkyl formate or with a cyanogen halide (followed, according to the nature of the compound of formula (I) which it is desired to obtain, by an alkylation reaction using an alkyl halide)
to yield a compound of formula (I/a), a particular case of the compounds of formula (I): 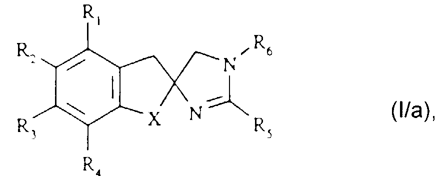 wherein X, R₁, R₂, R₃, R₄, R₅ and R₆ are as defined for formula (I),
which compound of formula (I/a)
is, if necessary, purified according to a customary purification technique,
is, if desired, separated into its isomers according to a customary purification technique,
and is optionally converted into its addition salts with a pharmaceutically acceptable acid.

9. Process for the preparation of compounds of formula (I) according to claim 1 wherein Y represents an oxygen or sulphur atom, characterised in that the compound of formula (VI) according to claim 8 is reacted with formic acid in an anhydrous medium saturated with hydrochloric acid
to yield a compound of formula (VII): 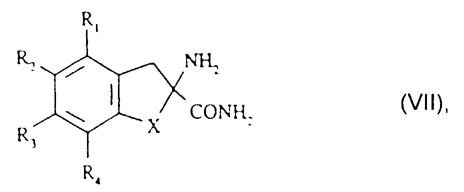 wherein R₁, R₂, R₃, R₄ and X are as defined for formula (I),
which is converted in a concentrated hydrochloric medium into a corresponding acid of formula (VIII): 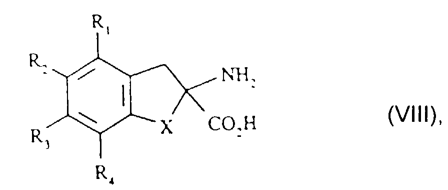 wherein R₁, R₂, R₃, R₄ and X are as defined for formula (I),
which is subjected to reduction with lithium aluminium hydride in an inert medium, to yield a compound of formula (IXa): 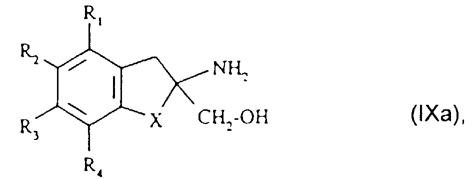 wherein R₁, R₂, R₃, R₄ and X are as defined for formula (I),
which compound of formula (IXa) is converted, according to the nature of the compounds of formula (I) which it is desired to obtain, into the corresponding tosylate using para-toluenesulphonic acid and then reacted with thiourea or thioacetic acid to yield, after hydrolysis, a compound of formula (IXb): 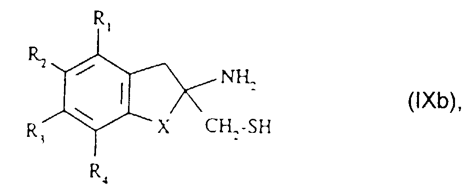 wherein R₁, R₂, R₃, R₄ and X are as defined for formula (I),
which compound of formula (IXa) or (IXb) is reacted with formamidine in an alcoholic medium, an alkyl formate or with a cyanogen halide (followed, according to the nature of the compound of formula (I) which it is desired to obtain, by an alkylation reaction using an alkyl halide)
to yield a compound of formula (I/b), a particular case of the compounds of formula (I): 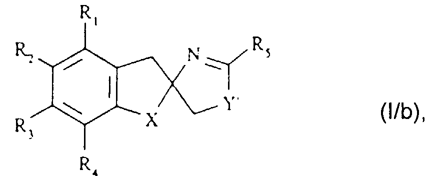 wherein R₁, R₂, R₃, R₄, R₅ and X are as defined hereinbefore and Y' represents an oxygen or sulphur atom,
which compound of formula (I/b)
is, if necessary, purified according to a customary purification technique,
is, if desired, separated into its isomers according to a customary purification technique,
and is optionally converted into its addition salts with a pharmaceutically acceptable acid.

10. Pharmaceutical compositions comprising as active ingredient at least one compound according to any one of claims 1 to 7 alone or in combination with one or more pharmaceutically acceptable, inert, non-toxic excipients or carriers.

11. Pharmaceutical compositions according to claim 10 comprising at least one active ingredient according to any one of claims 1 to 7 for use as a partial α₁- or α₂-adrenergic agonist in the treatment of venous disease and migraine.
